# EUROPEAN PATENT SPECIFICATION

(11) **EP 4 447 920 B1**
(45) Date of publication and mention of the grant of the patent: **23.07.2025**
(21) Application number: 22823459.7
(22) Date of filing: 30.11.2022
(51) Int. Cl.: A61K 8/44, A61K 8/34, A61K 8/891, A61K 8/36, A61K 8/26, A61Q 5/12

(54) **HAIR CONDITIONING COMPOSITION FOR IMPROVED DEPOSITION**
HAARPFLEGEZUSAMMENSETZUNG ZUR VERBESSERTEN ABSCHEIDUNG
COMPOSITION D'APRÈS-SHAMPOOING POUR DÉPÔT AMÉLIORÉ

(30) Priority: 16.12.2021 EP 21215037
(43) Date of publication of application: 23.10.2024
(73) Proprietor: Unilever IP Holdings B.V., 3013 AL Rotterdam (NL); Unilever Global IP Limited, Wirral, Merseyside CH62 4ZD (GB)
(72) Inventor: BARFOOT, Richard, Jonathan, 6708 WH Wageningen (NL); COOKE, Michael, James, 6708 WH Wageningen (NL); MENDOZA FERNANDEZ, Cesar, Ernesto, 6708 WH Wageningen (NL); SIMON, Amelie, Laura, 6708 WH Wageningen (NL)
(74) Representative: Chisem, Janet
(86) International application number: PCT/EP2022/083821
(87) International publication number: WO 2023/110402

(56) References cited:
- EP-B1- 1 534 222
- WO-A1-2020/126660
- US-B2- 8 530 399

## Description

### Field of the Invention

The invention is concerned with conditioning compositions, comprising a branched cationic surfactant in combination with fatty alcohol and a clay , for the treatment of bleached hair, which comprise a benefit agent to be deposited onto the hair during use and particularly relates to a conditioning composition that enables increased amounts of benefit agent to be deposited to bleached hair.

### Background and Prior art

In personal care compositions, such as hair treatment compositions, the deposition and delivery of benefit agents are often key drivers of product performance. For example, many of the hair conditioner products in the market today work to deliver benefits to hair by depositing benefit agents such as fragrance materials, silicones and damage repair actives onto the hair during wash and care processes.

However, consumers report being disappointed by the level of benefit derived from use of some compositions. This is usually caused by insufficient amount of benefit agents being delivered to the surface. It is, therefore, desirable to develop compositions that provide improved delivery of benefit materials to a surface, for example hair.

Bleached hair is known to be particularly poor at retaining silicones during and after application, leading to low levels of deposition and inadequate benefit to the user. The user, therefore, has to apply more product and may never reach the level of conditioning that is desired. Indeed, we have found that measured silicone levels delivered to bleached hair can be less than 20 % than that achieved for the same product used on virgin hair.

Various types of branched cationic compounds are known in hair treatment compositions for a variety of benefits.

Our own published applications WO 02/102334 and WO 01/43718 provide aqueous hair treatment compositions having cleansing and conditioning properties that comprise quaternary ammonium based cationic surfactants having defined hydrocarbyl chains.

Further, WO 2020/126377 A1 discloses compositions for improved deposition of benefit agents to hair. An example discloses a composition comprising (i) behentrimonoum chloride, (ii) cetearyl alcohol, (iii) an emulsified silicone and (iv) N,N,N-trimethyl-2-((2-octyldodecyl)oxy)-2-oxoethan-1-aminium methanesulphonate.

WO 2020/126659 A1 discloses a composition for improved benefit agent deposition onto hair, comprising: (i) 0.01 to 10 wt % of a linear, cationic conditioning surfactant; (ii) 0.1 to 10 wt % of a linear fatty material; (iii) a particulate benefit agent; (iv) 0.01 to 5 wt %, at 100 % active, of a branched cationic co-surfactant, as defined by a structure (1); wherein the molar ratios of branched cationic co-surfactants (iv) to linear cationic surfactants (i) are in the range of from 1:20 to 1:1; the compositions having a viscosity of 5,000 to 750,000 cp.

WO 2020/127542 A1 discloses a composition comprising: a) a conditioning base comprising: i) a cationic conditioning surfactant having from 16 to 32 carbon atoms; ii) a fatty alcohol having from 8 to 22 carbon atoms; and b) from 0.1 to 10 wt % of a conditioning silicone; (c) from 0.1 to 5 wt % of a diesterquat selected from a diesterquat that comprises branched, saturated chains, a diesterquat that comprises unbranched, unsaturated chains, and mixtures thereof; wherein the ratio ofb) to c) is from 1:1 to 1:0.1, to provide improved deposition of silicone on hair surfaces.

WO 2020/126660 A1 discloses a composition comprising: (i) 0.01 to 10 wt % of a linear, cationic conditioning surfactant; (ii) 0.1 to 10 wt % of a linear fatty material; (iii) a particulate benefit agent selected from conditioning actives, scalp actives, encapsulated fragrance, emulsified fragrance, and mixtures thereof; (iv) 0.01 to 5 wt %, at 100 % active, of a branched cationic co-surfactant, selected from structure 1, structure 2, structure 3 and mixtures thereof; wherein the molar ratios of branched cationic co-surfactants (iv) to linear cationic surfactants (i) are in the range of from 1:20 to 1:1 to provide improved particulate benefit agent deposition onto hair.

Despite the prior art, there remains a need to deliver improved delivery of benefit agents to bleached hair.

We have now surprisingly found that compositions comprising a combination of defined branched surfactants in combination with a fatty material, and a clay provide an unexpectedly large enhancement in the deposition of conditioning benefit agents (eg silicones) onto bleached hair.

All percentages quoted herein are by weight based on total weight, unless otherwise stated.

### Definition of the Invention

Accordingly, there is provided a composition comprising:
(i) 0.01 to 10 wt % of a branched cationic conditioning surfactant;
   selected from Structure 1: 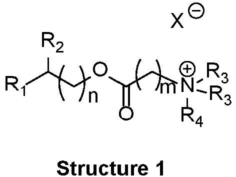
   wherein:
      - R₁ and R₂ comprise linear alkyl chains, saturated or unsaturated, with carbon-carbon chain lengths of from C₄ to C₂₀;
         - R₃ comprises an alkyl chain having a carbon-carbon chain length of from C₁ to C₄, preferably C₁ to C₂;
         - R₄ comprises a proton or an alkyl chain having a carbon-carbon chain length of from C₁ to C₄, preferably C₁ to C₂; and
      - n has a range of from 0 to 10;
      - m has a range of from 1 to 6, preferably selected from 1 and 2;
      - X^{Θ} is an organic or inorganic anion;
(ii) 0.1 to 10 wt % of a linear fatty material having at least one linear carbon-carbon chain, selected from a fatty alcohol, an alkoxylated fatty alcohol, a fatty acid and mixtures thereof;
(iii) 0.1 to 5 wt % of a clay; and
(iv) a particulate benefit agent selected from conditioning actives, wherein the conditioning actives are silicone emulsions;
wherein the molar ratios of branched cationic conditioning surfactants (i) to linear fatty material (ii) are in the range of from 1:20 to 1:1, preferably from 1:10 to 1:1, most preferably 1:5 to 1:2.

In a second aspect, the invention provides a method of increasing deposition of a particulate benefit agent selected from conditioning actives, which are silicone emulsions, to bleached hair comprising the step of applying to bleached hair a composition of the first aspect, compared with a similar composition that does not comprise a branched cationic conditioning surfactant in accordance with Structure 1, preferably compared with a composition that does not comprise branched cationic conditioning surfactants (i) and a linear fatty material (ii) in a molar ratio in the range of from 1:20 to 1:1, preferably from 1:10 to 1:1, most preferably 1:5 to 1:2.

The method of the invention preferably comprises an additional step of rinsing the composition from the bleached hair.

Preferably, the method is a method of increasing silicone deposition to bleached hair comprising the steps of applying to hair a composition as defined by the first aspect of the invention and rinsing the hair with water compared with a similar composition that does not comprise a branched cationic conditioning surfactant in accordance with Structure 1, preferably compared with a composition that does not comprise branched cationic conditioning surfactants (i) and a linear fatty material (ii) in a molar ratio in the range of from 1:20 to 1:1, preferably from 1:10 to 1:1, most preferably 1:5 to 1:2.

A third aspect provides a use of a composition of the first aspect to deliver increased amount of particulate benefit agent selected from conditioning actives, which are silicones, to bleached hair, compared with a similar composition that does not comprise a branched cationic conditioning surfactant in accordance with Structure 1, preferably compared with a composition that does not comprise branched cationic conditioning surfactants (i) and a linear fatty material (ii) in a molar ratio in the range of from 1:20 to 1:1, preferably from 1:10 to 1:1, most preferably 1:5 to 1:2.

Compositions in accordance with the invention are preferably formulated as conditioners for the treatment of hair (typically after shampooing) and subsequent rinsing.

### General description of the invention

Preferably, the treatment composition is selected from a rinse-off hair conditioner, a hair mask, a leave-on conditioner composition, and a pre-treatment composition, more preferably selected from a rinse-off hair conditioner, a hair mask, a leave-on conditioner composition, and a pre-treatment composition, for example an oil treatment, and most preferably selected from a rinse-off hair conditioner, a hair mask and a leave-on conditioner composition. The treatment composition is preferably selected from a rinse-off hair conditioner and a leave-on conditioner.

Rinse off conditioners for use in the invention are conditioners that are typically left on wet hair for 1 to 2 minutes before being rinsed off.

Hair masks for use in the present invention are treatments that are typically left on the hair for 3 to 10 minutes, preferably from 3 to 5 minutes, more preferably 4 to 5 minutes, before being rinsed off.

Leave-on conditioners for use in the invention are typically applied to the hair and left on the hair for more than 10 minutes, and preferably are applied to the hair after washing and not rinsed out until the next wash.

### The branched cationic conditioning surfactant

The branched cationic conditioning surfactant is present in an amount of from 0.01 to 10 wt %, preferably from 0.01 to 5 wt %, most preferably 0.1 to 2 wt % (at 100 % active and based on weight of total composition).

The molar ratio of branched cationic conditioning surfactants (i) to linear fatty material (ii) is in the range of from 1:20 to 1:1, preferably from 1:10 to 1:1, most preferably 1:5 to 1:2.

The branched cationic conditioning surfactant contains an ester group is selected from structure 1. 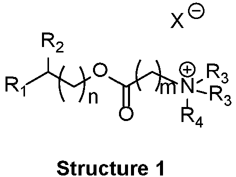 wherein:
- R₁ and R₂ comprise linear alkyl chains, saturated or unsaturated, with carbon-carbon chain lengths of from C₄ to C₂₀ preferably from C₆ to C₁₈;
   - R₃ comprises an alkyl chain having a carbon-carbon chain length of from C₁ to C₄, preferably C₁ to C₂;
   - R₄ comprises a proton or an alkyl chain having a carbon-carbon chain length of from C₁ to C₄, preferably C₁ to C₂; and
- n has a range of from 0 to 10, preferably selected from 0 and 1;
- m has a range of from 1 to 6, preferably selected from 1 and 2;
- X^{Θ} is an organic or inorganic anion;
wherein the molar ratios of branched cationic surfactants (i) to linear fatty material (ii) are in the range of from 1:20 to 1:1, preferably from 1:10 to 1:1, most preferably 1:5 to 1:2.

In structure 1, the amine head group is charged within the final formulation. Raw materials include, however, species where the charge is not permanent and can be induced by protonation in the formulation using a strong acid.

Optionally, at least one of R₁ and R₂, comprise linkages within the alkyl chain selected from the group consisting of an ester group (-OCO- or -COO-), an amido group (-NOC-or NCO-), and an ether group (-O-).

X^{Θ} is an organic or inorganic anion. Preferably, X^{Θ} comprises an anion selected from the halide ions; sulphates of the general formula RSO₃⁻, wherein R is a saturated or unsaturated alkyl radical having 1 to 4 carbon atoms, and anionic radicals of organic acids.

Preferred halide ions are selected from fluoride, chloride, bromide and iodide. Preferred anionic radicals of organic acids are selected from maleate, fumarate, oxalate, tartrate, citrate, lactate and acetate. Preferred sulphates are methanesulphonate and ethanesulphonate.

Most preferably, X^{Θ} comprises an anion selected from a halide, a methanesulfonate group and an ethanesulphonate group.

In a preferred embodiment,
- R₁ and R₂ comprise linear alkyl chains, saturated or unsaturated, with carbon-carbon chain lengths of from C₄ to C₂₀, preferably from C₆ to C₁₈;
- R₃ comprise either a proton or linear or branched alkyl chains, saturated or unsaturated, with carbon-carbon chain lengths of from C₁ to C₃
- n has a range of from 0 to 10, preferably selected from 0 and 1; and
- X^{Θ} is an organic or inorganic anion;

Methods for preparation of suitable branched cationic surfactants are known in the art and described, for example, in Chemistry, A European Journal, 2008, 14, 382. For example, 2-((2-octyldodecyl)oxy)-2-oxoethan-1-aminium methanesulphonate can be synthesized by the acid-catalysed condensation reaction of glycine with the specific guerbet alcohol, furnishing the desired product in one step.

Examples of suitable materials conforming to structure 1 are 2-((2-butyloctyl)oxy)-2-oxoethan-1-aminium methanesulphonate, 2-((2-hexyldecyl)oxy)-2-oxoethan-1-aminium methanesulphonate, 2-((2-octyldodecyl)oxy)-2-oxoethan-1-aminium methanesulphonate, 2-((2-decyltetradecyl)oxy)-2-oxoethan-1-aminium methanesulphonate, 2-((2-dodecylhexadecyl)oxy)-2-oxoethan-1-aminium methanesulphonate and 2-((2-tetradecyloctadecyl)oxy)-2-oxoethan-1-aminium methanesulphonate. Alternatively, a chloride counterion may be substituted for the methanesulphonate in the above examples.

### The fatty material

The composition of the invention comprises from 0.1 to 10 wt % of a linear fatty material. The combined use of fatty materials and cationic surfactants in conditioning compositions is believed to be especially advantageous, because this leads to the formation of a structured lamellar or liquid crystal phase, in which the cationic surfactant is dispersed.

Fatty materials comprise carbon-carbon chains. The term "linear" means that the carbon-carbon chains are linear in nature (ie free from branching). A "linear fatty material" has only linear carbon-carbon chains. The linear chains may be saturated or unsaturated.

The "linear fatty material" is selected from linear fatty alcohol, a linear alkoxylated fatty alcohol, a linear fatty acid and mixtures thereof. Preferably the linear fatty material is selected from a linear fatty alcohol and a linear fatty acid and mixtures thereof, most preferably a linear fatty alcohol.

Preferably, the alkyl chain of the fatty material is fully saturated. Representative fatty materials comprise from 8 to 22 carbon atoms, more preferably 16 to 22.

Suitable fatty alcohols comprise from 8 to 22 carbon atoms, preferably 16 to 22, most preferably C16 to C18. Fatty alcohols are typically compounds containing straight chain alkyl groups. Preferably, the alkyl groups are saturated. Examples of preferred fatty alcohols include cetyl alcohol, stearyl alcohol and mixtures thereof. The use of these materials is also advantageous in that they contribute to the overall conditioning properties of compositions for use in the invention.

Alkoxylated, (e.g. ethoxylated or propoxylated) fatty alcohols having from about 12 to about 18 carbon atoms in the alkyl chain can be used in place of, or in addition to, the fatty alcohols themselves. Suitable examples include ethylene glycol cetyl ether, polyoxyethylene (2) stearyl ether, polyoxyethylene (4) cetyl ether, and mixtures thereof.

The level of fatty material in conditioners of the invention is suitably from 0.1 to 10, and preferably from 0.1 to 5 percent by weight of the total composition.

### The Clay

The compositions of the invention comprise a clay. The clay is unmodified.

The clay is preferably selected from a bentonite clay and a hectorite clay, preferably a bentonite clay.

A suitable unmodified bentonite clay is available from Sigma. Also, under the tradename Bentone, available from Elementis.

The clay is present in an amount of from 0.1 to 10 wt %, preferably 0.1 to 5 wt %, most preferably from 0.5 to 4 %, by weight of the total composition.

We have found that the clay on its own does not cause silicone to deposit onto the surface of hair, but in combination with the branched cationic conditioning surfactant and fatty material of the present invention, high levels of silicone deposit onto hair.

### The particulate benefit agent

The composition of the invention comprises a particulate benefit agent. The particulate benefit agent is selected from conditioning actives, whereinthe particulate benefit agent is a silicone emulsion.

The total amount of particulate benefit agent is preferably from 0.1 wt % to 10 wt % of the total composition more preferably from 0.1 wt % to 5 wt %, even more preferably 0.25 wt % to 3 wt % and most preferably 0.25 to 1.5 wt %.

Preferred silicone emulsions do not comprise a hydrophobic modification, preferably the silicone emulsion is not a myristyloxyl modified silicone, most preferably not a myristyloxyl modified silicone or a cetyloxyl modified silicone. Most preferably, the silicone emulsions for use in the compositions of the invention are selected from emulsions of dimethicone, dimethiconol, amodimethicone and mixtures thereof.

Suitable emulsified silicones include polydimethylsiloxanes which have the CTFA designation dimethicone. Also suitable for use compositions of the invention are polydimethyl siloxanes having hydroxyl end groups, which have the CTFA designation dimethiconol. Preferably, the silicone is selected from the group consisting of dimethicone, dimethiconol, amodimethicone and mixtures thereof. Also preferred are blends of amino-functionalised silicones with dimethicones.

The viscosity of the emulsified silicone itself (not the emulsion or the final hair conditioning composition) is typically at least 10,000 cst at 25 °C the viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use in the compositions of the invention will typically have a D90 silicone droplet size in the composition of less than 30, preferably less than 20, more preferably less than 10 micron, ideally from 0.01 to 1 micron. Silicone emulsions having an average silicone droplet size (D50) of 0.15 micron are generally termed microemulsions.

Silicone particle size may be measured by means of a laser light scattering technique, for example using a 2600D Particle Sizer from Malvern Instruments.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation.

A further preferred class of silicones for inclusion in compositions of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone". A preferred amodimethicone is commercially available from Dow Corning as DC 7134.

Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex Dow Corning).

### Optional linear cationic conditioning surfactant

Compositions may comprise a linear cationic conditioning surfactant, which is cosmetically acceptable and suitable for topical application to the hair.

Preferably, the linear cationic conditioning surfactants have the formula 1: N⁺(R¹)(R²)(R³)(R⁴), wherein R¹, R², R³ and R⁴ are independently (C₁ to C₃₀) alkyl or benzyl.

In formula 1, preferably, one, two or three of R¹, R², R³ and R⁴ are independently (C₄ to C₃₀) alkyl and the other R¹, R², R³ and R⁴ group or groups are (C₁-C₆) alkyl or benzyl.

More preferably, one or two of R¹, R², R³ and R⁴ are independently (C₆ to C₃₀) alkyl and the other R¹, R², R³ and R⁴ groups are (C₁-C₆) alkyl or benzyl groups. Optionally, the alkyl groups may comprise one or more ester (-OCO- or -COO-), amido (-NOC- or NCO-), and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (e.g., oleyl). Alkyl groups are optionally ethoxylated on the alkyl chain with one or more ethyleneoxy groups.

Suitable quaternary amine salts for use in conditioner compositions according to the invention are quaternary amine salt comprising from 12 to 24 carbon atoms, preferably from 16 to 22 carbon atoms.

Suitable quaternary amine salts for use in conditioner compositions according to the invention include cetyltrimethylammonium chloride, behenyltrimethylammonium chloride, Behentrimonium methosulphate, BehenylAmido Propyl Di-Methyl Amine, cetyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, Stearalkonium Chloride, Stearalkonium methosulphate, didodecyldimethylammonium chloride, dioctadecyldimethylammonium chloride, tallowtrimethylammonium chloride. dihydrogenated tallow dimethyl ammonium chloride (e.g., Arquad 2HT/75 from Akzo Nobel) and cocotrimethylammonium chloride.

Preferred quaternary amine salts selected from behenyltrimethylammonium chloride, Behentrimonium methosulphate, cetyltrimethylammonium chloride, and mixtures thereof. A particularly useful cationic surfactant for use in conditioners according to the invention is cetyltrimethylammonium chloride, available commercially, for example as GENAMIN CTAC, ex Hoechst Celanese. Another particularly preferred cationic surfactant for use in conditioners according to the invention is behenyltrimethylammonium chloride, available commercially, for example as GENAMIN KDMP, ex Clariant.

Further suitable cationic surfactants include those materials having the CTFA designations Quaternium-5, Quaternium-31, and Quaternium-18. Mixtures of any of the foregoing materials may also be suitable.

Another example of a class of suitable cationic surfactants for use in the invention, either alone or together with one or more other cationic surfactants, is a combination of (i) and (ii) below:
(i) an amidoamine corresponding to the general formula (II):

   R¹CONH(CH₂)ₘN(R²)R³ (II)

   in which R¹ is a hydrocarbyl chain having 10 or more carbon atoms, R² and R³ are independently selected from hydrocarbyl chains of from 1 to 10 carbon atoms, and m is an integer from 1 to about 10; and
(ii) an acid.

As used herein, the term hydrocarbyl chain means an alkyl or alkenyl chain.

Preferred amidoamine compounds are those corresponding to formula (I) in which
R¹ is a hydrocarbyl residue having from about 11 to about 24 carbon atoms,
R² and R³ are each independently hydrocarbyl residues, preferably alkyl groups, having from 1 to about 4 carbon atoms, and m is an integer from 1 to about 4.

Preferably, R² and R³ are methyl or ethyl groups.

Preferably, m is 2 or 3, i.e. an ethylene or propylene group.

Preferred amidoamines useful herein include stearamido-propyldimethylamine (TAS), stearamidopropyldiethylamine, stearamidoethyldiethylamine, stearamidoethyldimethylamine, palmitamidopropyldimethylamine, palmitamidopropyl-diethylamine, palmitamidoethyldiethylamine, palmitamidoethyldimethylamine, behenamidopropyldimethyl-amine, behenamidopropyldiethylmine, behenamidoethyldiethyl-amine, behenamidoethyldimethylamine, arachidamidopropyl-dimethylamine, arachidamidopropyldiethylamine, arachid-amidoethyldiethylamine, arachidamidoethyldimethylamine, and mixtures thereof. Particularly preferred amidoamines useful herein are stearamidopropyldimethylamine, stearamidoethyldiethylamine, and mixtures thereof.

Commercially available amidoamines useful herein include:
stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England), and various amidoamines with tradenames SCHERCODINE series available from Scher (Clifton New Jersey, USA).

Acid may be any organic or mineral acid which is capable of protonating the amidoamine in the conditioner composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, lactic acid and mixtures thereof.

The primary role of the acid is to protonate the amidoamine in the hair treatment composition thus forming a (TAS) tertiary amine salt (stearamidopropyl dimethylamine) in situ in the hair treatment composition. The stearamidopropyl dimethylamine in effect is a non-permanent quaternary ammonium or pseudo-quaternary ammonium cationic surfactant.

Suitably, the acid is included in a sufficient amount to protonate more than 95 mole% (293 K) of the amidoamine present.

In conditioners for use in the invention, the level of linear cationic conditioning surfactant will generally range from 0.01 to 10%, more preferably 0.05 to 7.5%, most preferably 0.1 to 5% by total weight of cationic conditioning surfactant based on the total weight of the composition.

### Further Ingredients

The composition according to the invention may comprise any of a number of ingredients which are common to hair conditioning compositions.

Other ingredients may include, preservatives, colouring agents, polyols such as glycerine and polypropylene glycol, chelating agents such as EDTA, antioxidants such as vitamin E acetate, fragrances, antimicrobials and sunscreens. Each of these ingredients will be present in an amount effective to accomplish its purpose. Generally, these optional ingredients are included individually at a level of up to about 5% by weight of the total composition.

Preferably, the further ingredients include perfumes, preservatives, colours and conditioning silicones.

The compositions of the invention are preferably free from viscosity modifiers and thickening agents for example thickening polymers.

Mixtures of any of the above active ingredients may also be used.

Generally, such ingredients are included individually at a level of up to 2%, preferably up to 1%, by weight of the total composition.

Embodiments of the invention are given in the following examples, in which all percentages are quoted by weight based on total weight unless otherwise stated.

### Examples

### Example 1: Composition 1 in accordance with the invention, and comparative compositions A, B and C

The following compositions were prepared and are shown in Table 1:
Composition 1: comprises an unmodified bentonite clay in combination with the branched cationic surfactant and fatty alcohol, in accordance with the invention.
Compositions A and B: comprise clay without the branched cationic surfactant and fatty alcohol.
Composition C comprises branched cationic surfactant and fatty alcohol but no clay.

**Table 1: Composition of Composition 1 in accordance with the invention, and comparative compositions A, B and C**

| **Ingredient** | **Trade/INCI name** | **Amount (wt %)** | | | |
|---|---|---|---|---|---|
| | | **A** | **B** | **C** | **1** |
| Branched cationic surfactant | N,N,N-Trimethyl-2-((2-octyldodecyl)oxy)-2-oxoethan-1-aminium methanesulfonate | - | - | 1.85 | 2.2 |
| Fatty Alcohol | Ginol 1618 TA | - | - | 4.15 | 4.15 |
| Clay | Bentonite clay (unmodified) | 5 | 10 | 0 | 2 |
| Silicone | Xiameter MEM-7134 | 1.43 | 1.43 | 1.43 | 1.43 |
| Fragrance | Perfume | 0.6 | 0.6 | 0.6 | 0.6 |
| Water | | To 100 | To 100 | To 100 | To 100 |

Formulations were made by adding the cationic surfactant to the fatty alcohol and stirring at about 85°C before gradually adding this mixture to water, typically at 55°C, and mixing at 60°C. The mixture was then cooled to ambient temperature by adding more water. Clay was then added as well as other remaining ingredients and the final mixture stirred.

### Example 2: Treatment of hair with compositions 1, A, B and C

The hair (virgin) used was dark brown European hair, in switches of 5 g weight and 6 inches in length.

Bleaching of virgin hair was carried out using by applying Platine Precision White Compact Lightening Powder (L'Oreal Professionnel Paris, Paris, France) mixed with 9% cream peroxide, 30 'vol' (Excel GS Ltd, UK) (60g of powder mixed with 120g cream peroxide) and leaving for 30 minutes. Hair was then rinsed with water for 2 minutes.

Virgin or bleached hair was first treated with a cleansing shampoo using the following method:-
The hair fibres were held under running water for 30 seconds, shampoo applied at a dose of 0.1 ml of shampoo per 1g of hair and rubbed into the hair for 30 seconds. Excess lather was removed by holding under running water for 30 seconds and the shampoo stage repeated. The hair was rinsed under running water for 1 minute.

The wet hair (virgin or bleached) was then treated with the compositions using the following method:-
Conditioner was applied to the wet hair at a dose of 0.2 ml of conditioner per 1g of hair and massaged into the hair for 1 minute. The hair was rinsed under running water for 1 minute and excess water removed.

The amount of silicone deposited onto the hair was then determined using XRF.

### Example 3: Silicone Deposition onto bleached hair treated with Compositions 1, A and B

**Table 2: Deposition of silicone onto bleached hair by Compositions 1 in accordance with the invention and comparative compositions A, B and C**

| **Composition** | **Silicone ppm** | **STDEV ppm** |
|---|---|---|
| 1 | 2135 | 329 |
| A | 28 | 90 |
| B | -31 | 92 |
| C | 183 | 28 |

It will be seen that the clay on its own does not cause silicone to deposit onto the surface of hair, but in combination with the branched cationic conditioning surfactant and fatty material of the present invention, high levels of silicone deposit onto hair.

## Claims

1. A composition comprising:
(i) 0.01 to 10 wt % of a branched cationic conditioning surfactant;
selected from structure 1, 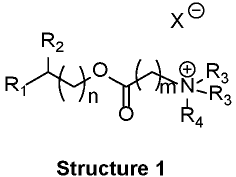
wherein:
• R₁ and R₂ comprise linear alkyl chains, saturated or unsaturated, with carbon-carbon chain lengths of from C₄ to C₂₀;
• R₃ comprises an alkyl chain having a carbon-carbon chain length of from C₁ to C₄, preferably C₁ to C₂;
• R₄ comprises a proton or an alkyl chain having a carbon-carbon chain length of from C₁ to C₄, preferably C₁ to C₂; and
• n has a range of from 0 to 10;
• m has a range of from 1 to 6, preferably selected from 1 and 2;
(ii) 0.1 to 10 wt % of a linear fatty material comprising linear carbon-carbon chains, selected from a fatty alcohol, an alkoxylated fatty alcohol, a fatty acid and mixtures thereof;
(iii) 0.1 to 10 wt % of a clay; and
(iv) a particulate benefit agent selected from conditioning actives, wherein the conditioning actives are silicone emulsions;
wherein the molar ratio of branched cationic surfactants (i) to linear fatty material (ii) is in the range of from 1:20 to 1:1.

2. A composition as claimed in claim 1, wherein the clay is a bentonite clay.

3. A composition as claimed in claim 2, wherein the silicone emulsions are selected from emulsions of dimethicone, dimethiconol, amodimethicone and mixtures thereof.

4. A composition as claimed in claim 2 or claim 3, wherein the silicone emulsions do not comprise a hydrophobic modification and preferably are not a myristyloxyl modified silicone.

5. A composition as claimed in any preceding claim, wherein the particulate benefit agent is present in an amount of from 0.1 wt % to 10 wt % of the total composition.

6. A composition as claimed in claim 5, wherein the particulate benefit agent is present in an amount of from 0.25 to 1.5 wt %.

7. A composition as claimed in any preceding claim, wherein X⁻ comprises an anion selected from a halide, a methanesulfonate group and an ethanesulphonate group.

8. A composition as claimed in any preceding claim, wherein the molar ratio of branched cationic surfactants (i) to linear fatty material (ii) is in the range of from 1:10 to 1:1.

9. A composition as claimed in any preceding claim, wherein the branched cationic conditioning surfactant is present in an amount of from 0.01 to 5 wt %.

10. A method of increasing deposition of a particulate benefit agent selected from conditioning actives, wherein the conditioning actives are silicone emulsionsto bleached hair comprising the steps of applying to hair a composition as defined in any of claims 1 to 9 and rinsing the hair with water, compared with a similar composition that does not comprise a branched cationic conditioning surfactant in accordance with Structure 1.

11. A use of a composition defined by any of claims 1 to 9 to deliver increased amount of particulate benefit agent selected from conditioning actives, wherein the conditioning actives are silicone emulsions, to bleached hair, compared with a similar composition that does not comprise a branched cationic conditioning surfactant in accordance with Structure 1.

## Patentansprüche

1. Zusammensetzung, umfassend:
(i) 0,01 bis 10 Gew.-% eines verzweigten kationischen
Konditionierungstensids; ausgewählt aus Struktur 1, 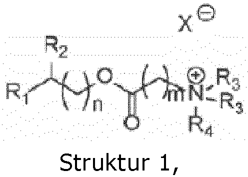
worin:
• R₁ und R₂ lineare Alkylketten, gesättigt oder ungesättigt, mit Kohlenstoff-Kohlenstoff-Kettenlängen von C₄ bis C₂₀ umfassen;
• R₃ eine Alkylkette mit einer Kohlenstoff-Kohlenstoff-Kettenlänge von C₁ bis C₄, vorzugsweise von C₁ bis C₂, umfasst;
• R₄ ein Proton oder eine Alkylkette mit einer Kohlenstoff-Kohlenstoff-Kettenlänge von C₁ bis C₄, vorzugsweise von C₁ bis C₂, umfasst; und
• n einen Bereich von 0 bis 10 aufweist;
• m einen Bereich von 1 bis 6 aufweist, vorzugsweise ausgewählt aus 1 und 2,;
(ii) 0,1 bis 10 Gew.-% eines linearen Fettmaterials, das lineare Kohlenstoff-Kohlenstoff-Ketten umfasst, ausgewählt aus einem Fettalkohol, einem alkoxylierten Fettalkohol, einer Fettsäure und Mischungen davon;
(iii) 0,1 bis 10 Gew.-% eines Tons; und
(iv) ein teilchenförmiges Pflegemittel, ausgewählt unter Konditionierungswirkstoffen, wobei die Konditionierungswirkstoffe Silikonemulsionen darstellen;
wobei das Molverhältnis von verzweigten kationischen Tensiden (i) zu linearem Fettmaterial (ii) in dem Bereich von 1:20 bis 1:1 liegt.

2. Zusammensetzung, wie im Anspruch 1 beansprucht, wobei der Ton ein Bentonitton ist.

3. Zusammensetzung, wie im Anspruch 2 beansprucht, wobei die Silikonemulsionen unter Emulsionen von Dimethicon, Dimethiconol, Amodimethicon und Mischungen davon ausgewählt sind.

4. Zusammensetzung, wie im Anspruch 2 oder Anspruch 3 beansprucht, wobei die Silikonemulsionen keine hydrophobe Modifikation umfassen und vorzugsweise kein Myristyloxyl-modifiziertes Silikon sind.

5. Zusammensetzung, wie in einem vorhergehenden Anspruch beansprucht, wobei das teilchenförmige Pflegemittel in einer Menge von 0,1 Gew.-% bis 10 Gew.-% der gesamten Zusammensetzung vorliegt.

6. Zusammensetzung, wie im Anspruch 5 beansprucht, wobei das teilchenförmige Pflegemittel in einer Menge von 0,25 bis 1,5 Gew.-% vorliegt.

7. Zusammensetzung, wie in einem vorhergehenden Anspruch beansprucht, wobei X⁻ ein Anion umfasst, ausgewählt unter einem Halogenid, einer Methansulfonatgruppe und einer Ethansulfonatgruppe.

8. Zusammensetzung, wie in einem vorhergehenden Anspruch beansprucht, wobei das Molverhältnis der verzweigten kationischen Tenside (i) zum linearen Fettmaterial (ii) in dem Bereich von 1:10 bis 1:1 liegt.

9. Zusammensetzung, wie in einem vorhergehenden Anspruch beansprucht, wobei das verzweigte kationische Konditionierungstensid in einer Menge von 0,01 bis 5 Gew.-% vorliegt.

10. Verfahren zur erhöhten Abscheidung eines partikelförmigen Pflegemittels, ausgewählt unter Konditionierungswirkstoffen, wobei die Konditionierungswirkstoffe Silikonemulsionen sind, auf gebleichtem Haar, umfassend die Schritte des Auftragens einer Zusammensetzung, wie in einem der Ansprüche 1 bis 9 definiert, auf Haar und Spülen des Haars mit Wasser, im Vergleich mit einer ähnlichen Zusammensetzung, die kein verzweigtes kationisches Konditionierungstensid gemäß Struktur 1 enthält.

11. Verwendung einer Zusammensetzung, definiert in einem der Ansprüche 1 bis 9, zur Abgabe einer erhöhten Menge eines partikelförmigen Pflegemittels, ausgewählt unter Konditionierungswirkstoffen, wobei die Konditionierungswirkstoffe Silikonemulsionen sind, auf gebleichtes Haar, im Vergleich mit einer ähnlichen Zusammensetzung, die kein verzweigtes kationisches Konditionierungstensid gemäß Struktur 1 enthält.

## Revendications

1. Composition comprenant:
(i) 0,01 à 10 % en poids d'un tensioactif conditionneur cationique ramifié; choisi dans la structure 1, 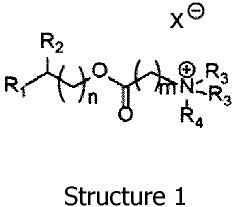 dans laquelle:
• R₁ et R₂ comprennent des chaînes alkyles linéaires, saturées ou insaturées, avec des longueurs de chaîne carbone-carbone de C₄ à C₂₀;
• R₃ comprend une chaîne alkyle ayant une longueur de chaîne carbone-carbone de C₁ à C₄, de préférence de C₁ à C₂;
• R₄ comprend un proton ou une chaîne alkyle ayant une longueur de chaîne carbone-carbone de C₁ à C₄, de préférence de C₁ à C₂; et
• n a une plage de 0 à 10;
• m a une plage de 1 à 6, de préférence choisi parmi 1 et 2;
(ii) 0,1 à 10 % en poids d'une matière grasse linéaire comprenant des chaînes carbone-carbone linéaires, choisie parmi un alcool gras, un alcool gras alcoxylé, un acide gras et leurs mélanges;
(iii) 0,1 à 10 % en poids d'une argile; et
(iv) un agent bénéfique particulaire choisi parmi les actifs conditionneurs, où les actifs ceonditionneurs sont des émulsions de silicone;
dans laquelle le rapport molaire des tensioactifs cationiques ramifiés (i) à la matière grasse linéaire (ii) est dans la plage de 1:20 à 1:1.

2. Composition selon la revendication 1, dans laquelle l'argile est une argile bentonite.

3. Composition selon la revendication 2, dans laquelle les émulsions de silicone sont choisies parmi les émulsions de diméthicone, diméthiconol, amodiméthicone et leurs mélanges.

4. Composition selon la revendication 2 ou la revendication 3, dans laquelle les émulsions de silicone ne comprennent pas de modification hydrophobe et, de préférence, ne sont pas un silicone modifié par myristyloxyle.

5. Composition selon l'une quelconque des revendications précédentes, dans laquelle l'agent bénéfique particulaire est présent en une quantité de 0,1 % en poids à 10 % en poids de la composition totale.

6. Composition selon la revendication 5, dans laquelle l'agent bénéfique particulaire est présent en une quantité de 0,25 à 1,5 % en poids.

7. Composition selon l'une quelconque des revendications précédentes, dans laquelle X⁻ comprend un anion choisi parmi un halogénure, un groupe méthanesulfonate et un groupe éthanesulfonate.

8. Composition selon l'une quelconque des revendications précédentes, dans laquelle le rapport molaire des tensioactifs cationiques ramifiés (i) à la matière grasse linéaire (ii) est dans la plage de 1:10 à 1:1.

9. Composition selon l'une quelconque des revendications précédentes, dans laquelle le tensioactif conditionneur cationique ramifié est présent en une quantité de 0,01 à 5 % en poids.

10. Procédé d'augmentation du dépôt d'un agent bénéfique particulaire choisi parmi les actifs conditionneurs, où les actifs conditionneurs sont des émulsions de silicone, sur des cheveux décolorés, comprenant les étapes d'application sur les cheveux d'une composition telle que définie dans l'une quelconque des revendications 1 à 9 et de rinçage des cheveux à l'eau, comparée à une composition similaire qui ne comprend pas de tensioactif conditionneur cationique ramifié selon la Structure 1.

11. Utilisation d'une composition définie par l'une quelconque des revendications 1 à 9 pour délivrer une quantité accrue d'un agent bénéfique particulaire choisi parmi les actifs conditionneurs, dans laquelle les actifs conditionneurs sont des émulsions de silicone, à des cheveux décolorés, comparée à une composition similaire qui ne comprend pas de tensioactif revitalisant cationique ramifié selon la Structure 1.
